# EUROPEAN PATENT APPLICATION

(11) **EP 2 210 596 A1**
(43) Date of publication of application: **28.07.2010**
(21) Application number: 09382008.2
(22) Date of filing: 22.01.2009
(51) Int. Cl.: A61K 9/20, A61K 9/36, A61K 31/663

(54) **Pharmaceutical composition of ibandronate sodium salt or a hydrate thereof**

(71) Applicant: Laboratorios Liconsa, S.A., 08028 Barcelona (ES)
(72) Inventor: Jurado Sánchez, Francisco, 19200, Azuqueca de Henares (Guadalajara) (ES); Rizo Martínez, José Miguel, 28043, MADRID (ES); Arroyo Hidalgo, Sergio, 28011, Madrid (ES); Manjón Cembellín, Óscar, 28022, Madrid (ES)
(74) Representative: ZBM Patents

(57) **Abstract**

The present invention comprises a pharmaceutical composition of Ibandronate sodium salt or a hydrate thereof which comprises microcrystalline cellulose with a median particle size (D50) from 30 to 150 µm and croscarmellose sodium from 10 to 15% by weight, a tablet which comprises this pharmaceutical composition and a process for its preparation.

## Description

### FIELD OF THE INVENTION

The present invention relates to a pharmaceutical composition of Ibandronate sodium salt, to tablets containing it, and to their preparations processes.

### BACKGROUND ART

Ibandronate sodium is the International Nonproprietary Name (INN) of [3-(*N-*methyl-*N*'-pentyl)-amino-1-hydroxypropane-1,1-diphosphonic acid monosodium salt. Ibandronate sodium is a third-generation nitrogen-containing bisphosphonate derivative characterized by an aliphatic tertiary amine side chain.

The structure of Ibandronate sodium corresponds to formula (I):

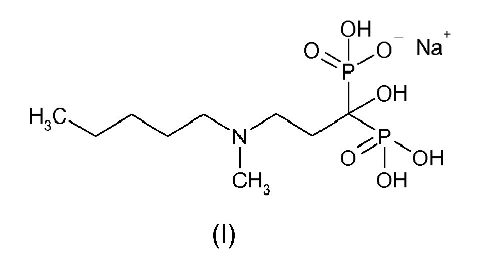

It was first disclosed in US patent 4,927,814. It is marketed under the name of Bonviva® for the treatment of bone disorders such as hypercalcaemia of malignancy, osteolysis, Pag>et's disease, osteoporosis, and metastatic bone disease. Bonviva® has a rapid dissolution profile where more than 60% of free Ibandronic acid is dissolved up to 5 minutes and more than 85% up to 15 minutes (cf. figure 3).

A suitable application of the active ingredient is its administration on one, two or three consecutive days per month. For instance, international patent application WO 03/95029 discloses an intermittent regimen comprising the administration of at least 120% of the expected efficacious daily dose of bisphosphonate and one or more pharmaceutically acceptable excipients, for the prevention or treatment of disorders characterise by pathologically increased bone resorption.

Granulation compositions of bisphosphonates are known in the art (cf. international patent application WO 94/12200). However, the use of granulation techniques for preparing pharmaceutical compositions of Ibandronate sodium may produce the degradation of active ingredient. Generally, the degradation of bisphosphonic acid is particularly pronounced in the presence of water and/or elevated temperature. It is supposed that this incompatibility is due to the reaction of the free amino group of the bisphosphonic acid with the glycosilic alcohol of a sugar, for example the binder. This reaction results in the formation of brown pigmented degradates and as a consequence the decrease of the effective dose of the active ingredient in the pharmaceutical composition.

Direct compression formulations have also been formulated in order to avoid the degradation of the active ingredient during granulation processes and tabletting. For instance, international patent application WO 94/12200 discloses that preparing a pharmaceutical composition of Ibandronate sodium in the presence of lactose by direct compression, a pharmaceutical formulation where the bisphosponic acid is stable can be obtained.

Ibandronic acid and its salts must be administered over a long periods of time, therefore, oral pharmaceutical compositions are more accepted for patients. However, oral treatment is complicated because of the low oral tolerability of bisphosphonic acid and particularly, aminobisphosphonic acid and its salts, which causes diseases of the upper gastrointestinal tract, such as gastrointestinal reflux disorders, esophagitis, dyspepsia and ulcers. It is also known that motility disorders may occur when swallowing the tablets to raise odynophagia or oesophageal strictures. Frequently, this is the case of elderly patients or those patients that are forced to take bisphosphonic acids in a lying position.

Thus, independently of the manufacturing process of the pharmaceutical composition is important to have a rapid dissolution profile in order to achieve a high concentration of the active ingredient in the stomach at short time to avoid gastrointestinal side effects.

European patent application EP 936.913 discloses another orally coated form of Ibandronate with an adjuvant coating or film-coating to reduce the gastrointestinal side effects of bisphosphonate. In this case, the film-coating is dissolved in a short period of time independently of the pH value. Thus, the active ingredient is dissolved in the stomach achieving a high local concentration. It is disclosed that the rapid dissolution profile of the active ingredient is due to the properties of the film-coating regardless of the composition of the core.

From what is known in the art it is derived that there is still the need of providing stable pharmaceutical compositions of Ibandronate sodium salt where the active ingredient has a rapid dissolution profile.

### SUMMARY OF THE INVENTION

Inventors have found that a pharmaceutical composition of Ibandronate sodium salt or a hydrate thereof which comprises microcrystalline cellulose with a median particle size (D50) from 30 to 150 µm and croscarmellose sodium in an amount from 10 to 15% by weight of the total weight of the pharmaceutical composition, has an appropriate dissolution profile and also shows a good stability, being also advantageous because of it can be manufactured by a simple process. Moreover, the rapid dissolution of the active ingredient from the pharmaceutical composition decreases the gastrointestinal side effects when Ibandronate sodium salt is orally administered.

Thus, an aspect of the present invention refers to the provision of a pharmaceutical composition which comprises from 0.5 to 40% by weight of Ibandronate sodium salt, from 15 to 30% by weight of microcrystalline cellulose with a median particle size (D50) from 30 to 150 µm and from 10 to 15% by weight of croscarmellose sodium.

Another aspect of the present invention relates to a process for the preparation of the pharmaceutical composition as defined above, which comprises: (a) mixing Ibandronate sodium salt with microcrystalline cellulose, a diluent, and croscarmellose sodium; (b) incorporating a lubricant to the mixture of step (a); and (c) incorporating an anti-adherent to the mixture of step (b).

### BRIEF DESCRIPTION OF THE DRAWINGS

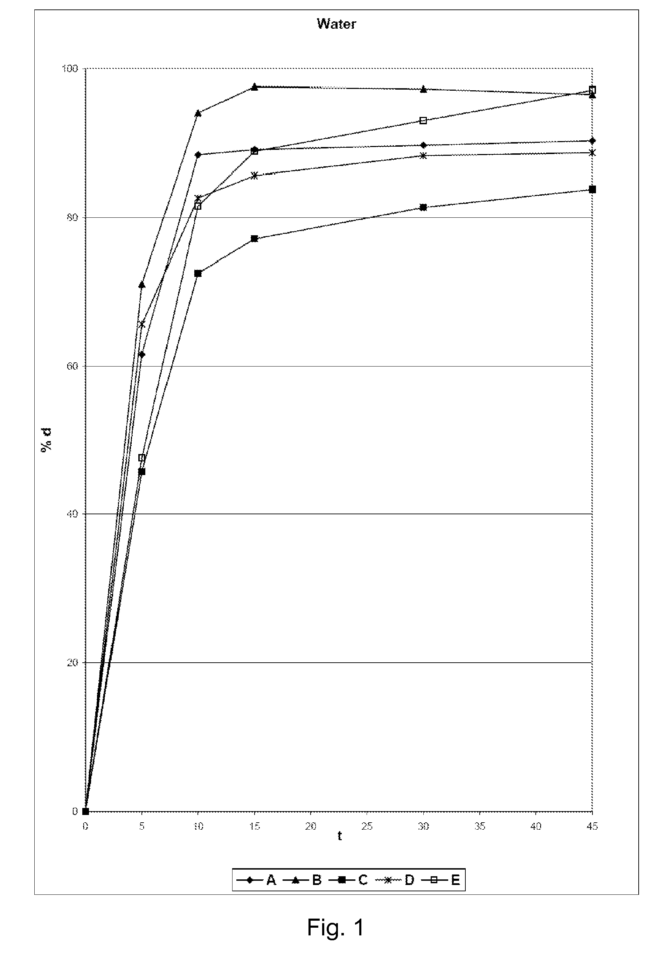

FIG. 1 shows a comparative scheme of the dissolution profile between the marketed product (Bonviva®) and the pharmaceutical compositions of the present invention. The dissolution tests fulfil the requirements of the Food and Drug Administration (FDA). Units of the scheme are as follows: d is the percentage of active ingredient dissolved in water and t is time in minutes. The description of the percentages of croscarmellose and median particle size of microcrystalline cellulose of the pharmaceutical compositions is as follows: A comprises Avicel PH 102 and 15% croscarmellose; B is Bonviva® 150mg Lot. B1064; C comprises Avicel PH 102 and 5% croscarmellose; D comprises Avicel PH 102 and 10% croscarmellose and E comprises comprecel 200 (microcrystalline cellulose) and 10% croscarmellose.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "median particle size" refers to a median diameter or medium value of particle diameter. Thus, the 50% of particles are larger than the reference value and the other 50% are smaller.

The term "lubricant" refers to a material which prevents adhesion of the tablet material to the surface of the dies and punches, reduces interparticle friction, facilitates the ejection of the tablets from the die cavity, and may improve the rate of flow of the tablet granulation. Commonly used lubricant includes talc, magnesium stearate, calcium stearate, stearic acid, glyceryl behanate, hydrogenated vegetable oils, and polyethylene glycol (PEG).

The term "anti-adherent agent" refers to a material which reduces the adhesion between the powder and the punch faces and thus prevents sticking to the tablet punches.

The term "tablet" refers to a compressed pharmaceutical dosage formulation of all shapes and sizes, whether coated or uncoated. In case of coated tablet, substances which may be used for coating tablets include, for instance, hydroxypropylmethylcellulose, hydroxypropylcellulose, titanium oxide, talc, sweeteners, and colorants.

The term "superdisintegrant agent" refers to a type of disintegrant that swells from four to forty fold in less than 30 seconds when placed in an aqueous medium. Superdisintegrants commonly used are Veegum HV, methylcellulose, agar, bentonite, cellulose and wood products, natural sponge, pectin, cation exchange resins, alginic acid, guar gum, citrus pulp, starch, and carboxymethylcellulose. Preferred superdisintegrants are croscarmellose sodium, crospovidone, sodium starch glycolate or mixtures thereof.

The term "percentage (%) by weight" refers to the percentage of each ingredient of the pharmaceutical composition in relation to the total weight of the tablet.

As mentioned above, an aspect of the present invention refers to a pharmaceutical composition of Ibandronate sodium salt or a hydrate thereof which comprises microcrystalline cellulose with a median particle size (D50) from 30 to 150 µm and from 10 to 15% by weight of croscarmellose sodium.

It is known that the particle size of some excipients, for example microcrystalline cellulose, could modify the physical properties of a pharmaceutical composition. Generally, an increase in the particle size would cause as well a greater fluidity of the powder blend in direct compression processes as would avoid weight variations and homogenisation problems. On the other hand, croscarmellose sodium can be considered a superdisintegrant. It is known that a superdisintegrant agent is commonly used in the manufacturing of tablets. This name comes from the low levels (2-4%) at which they are completely effective. In particular, croscarmellose sodium is used in tablet formulations, as well in direct compression as wet granulation processes. Generally, concentrations of up to 5% w/w of croscarmellose sodium are used as a tablet disintegrant, although normally 2% w/w is used in tablets prepared by direct compression (cf. Handbook of Pharmaceutical Excipients. Kibbe, Arthur H, Pharmaceutical Press. 3rd edition, pp.160).

However, in spite of these teachings, it has been found that the required dissolution profile of the Ibandronate sodium salt or a hydrate thereof, can be achieved by a tablet comprising microcrystalline cellulose having a specific median particle size (D50), in particular from 30 to 150 µm, together with croscarmellose sodium in an amount from 10 to 15%. This specific selection of excipients allows preparing pharmaceutical compositions in form of tablets by direct compression, which is advantageous by its simplicity.

The target dissolution profile comprises that more than 60% of free Ibandronic acid is dissolved up to 5 minutes and more than 85% up to 15 minutes (cf. figure 1). Moreover, the high concentration of active ingredient in the stomach also reduces the gastrointestinal side effects.

In a particular embodiment, the Ibandronate sodium salt is a monohydrate. In another particular embodiment, the Ibandronate sodium salt is a hemihydrate.

In a preferred embodiment, the median particle size (D50) of microcrystalline cellulose is 100 µm. It means that the 50% of particles of microcrystalline cellulose are smaller than 100 µm.

In another preferred embodiment, the pharmaceutical composition comprises from 11 to 15% by weight of croscarmellose sodium. In a more preferred embodiment, the composition comprises from 12 to 15% by weight of croscarmellose sodium. In a particular embodiment the composition comprises from 13 to 15% by weight of croscarmellose sodium. In another particular embodiment the composition comprises from 14 to 15% by weight of croscarmellose sodium. In another more preferred embodiment the composition comprises 15% by weight of croscarmellose sodium.

In a preferred embodiment, the pharmaceutical composition comprises 36% by weight of Ibandronate sodium salt or a hydrate thereof. The percentage of 36% by weight of Ibandronate sodium salt can represent between 50-150 mg of free Ibandronic acid in relation to the total weight of the tablet. This quantity of the free active ingredient is suitable for its administration daily to 50 mg or once per month to 150 mg. The intermittent regimen, that is once monthly, may improve the level of compliance of the treatment, as well as management and patient satisfaction. Thus, in a more preferred embodiment the pharmaceutical composition is administered once per month. In a particular embodiment, the pharmaceutical composition is administered once daily.

In another preferred embodiment, the pharmaceutical composition further comprises a diluent selected from the group consisting of anhydrous lactose, hydrous fast flow lactose, calcium carbonate, dibasic calcium phosphate, dextrose, fructose, isomaltose. magnesium carbonate, magnesium oxide, mannitol, maltose, sorbitol, sodium alginate, sucrose and xylitol. In a more preferred embodiment the diluent is selected from anhydrous lactose and hydrous fast flow lactose. Both diluents are widely used in direct compression of tablets. In another preferred embodiment, the pharmaceutical composition further comprises a lubricant and an anti-adherent agent.

In a more preferred embodiment, the pharmaceutical composition comprises: 36.7% by weight of Ibandronate sodium salt or a hydrate thereof; 25.3% by weight of microcrystalline cellulose; 14.7% by weight of croscarmellose sodium; 19.3% by weight fast flow lactose; 1 % by weight of magnesium stearate; and 1 % by weight of colloidal anhydrous silica.

The pharmaceutical composition of the invention is especially appropriate for the preparation of tablets. In particular, for the preparation of tablets by direct compression. The tablets can be film-coating tablets. In a particular embodiment, the film-coating comprises hydroxypropylcellulose, titanium dioxide, and polyethylenglycol 6000. This film-coating is independently of the pH value so; a high concentration of free Ibandronic acid is achieved in a short period of time, obtaining the required dissolution profile.

In a particular embodiment, the pharmaceutical composition comprises from 0.5 to 40% by weight of Ibandronate sodium salt or a hydrate thereof, from 15 to 30% by weight of microcrystalline cellulose with a median particle size (D50) from 30 to 150 µm and from 10 to 15% by weight of croscarmellose sodium, and is manufactured by direct compression to obtain a tablet. The dissolution profile of these tablets comprises that more than 60% of free Ibandronic acid is dissolved up to 5 minutes and more than 85% up to 15 minutes.

Monohydrate Ibandronate sodium salt is commercially available as Bonviva®. From the list of excipients of the technical data sheet of Bonviva®, it follows that the process for the manufacturing of the corresponding film-coating tablet may imply a granulation process. Due to the instability of the Ibandronate sodium salt in the presence of wetting agents, such as water, and/or elevated temperature, excipients and conditions commonly used in wet granulation and dry granulation, it is desirable to be able to formulate the Ibandronate sodium salt by direct compression, avoiding degradation of the active ingredient and the need of a precise control of the formulation conditions when dry or wet granulation techniques are used. The specific selection of excipients of the present invention allows formulating the tablets by direct compression which is advantageous for the reasons mentioned above.

Thus, direct compression is preferably used in the manufacturing of the tablets of the present invention. The direct compression process of the present invention requires only blending the ingredients in an appropriate order without granulation and avoids the use of binders, water and/or high temperatures, wherein the Ibandronate sodium salt could degrade. The stability of the active ingredient in the pharmaceutical formulation of the present invention was confirmed with a three-month data stability test (cf. example 4).

Thus, the pharmaceutical compositions of the invention can be prepared by a process which comprises: (a) mixing Ibandronate sodium salt or a hydrate thereof with microcrystalline cellulose, a diluent, and croscarmellose sodium; (b) incorporating a lubricant to the mixture of step (a); and (c) incorporating an anti-adherent to the mixture of step (b).

In a preferred embodiment, the Ibandronate sodium salt or a hydrate thereof is previously sieved through a sieve of 813 µm; the lubricant and the anti-adherent are sieved through a sieve of 600 µm.

Throughout the description and claims the word "comprise" and variations of the word, are not intended to exclude other technical features, additives, components, or steps.

Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

In the following, the present invention shall be illustrated by means of some examples, which are not construed to be viewed as limiting the scope of the invention.

### Example 1: Process for manufacturing a film-coating tablet by direct compression

The tablet composition is as follow:

**Core Tablet**

| **Ingredients** | **Per tablet (mg)** | **Per tablet (%)*** | **Per 3268 tablets (mg)** |
|---|---|---|---|
| Monohydrate Ibandronate sodium | 168.75 | 36.76 | 534.30 |
| Lactose fast-flow | 88.75 | 19.34 | 323.55 |
| Microcrystalline cellulose. Avicel PH 102 | 116.10 | 25.29 | 387.15 |
| Croscarmellose sodium | 67.50 | 14.70 | 225.00 |
| Magnesium stearate | 4.50 | 0.98 | 15.00 |
| colloidal anhydrous silica | 4.50 | 0.98 | 15.00 |

**Film-coating**

| **Ingredients** | **Per tablet (mg)** | **Per tablet (%)*** | **Per 900 (g) of core (g)** |
|---|---|---|---|
| Hydroxypropylcellulose | 5.00 | 1.09 | 10.0 |
| Titanium dioxide | 2.40 | 0.52 | 4.7 |
| Macrogol (PEG 6000) | 1.50 | 0.32 | 2.9 |

| | | | |
|---|---|---|---|
| *The percentage of each ingredient is referred to the total weight of the tablet. | | | |

The core weight is 450 mg and the total weight is 465mg, the amount of active ingredient per tablet is equivalent to 150 mg of free Ibandronic acid.

### Process for manufacturing of the core tablet

Lactose fast-flow, microcrystalline cellulose, sieved monohydrate Ibandronate sodium salt (equivalent to 150 mg free Ibandronic acid per tablet) thought a sieve of 813 µm and sodium croscarmellose were consecutively added to a blender. The mixture was blended for 12 minutes at 13 rpm. Colloidal anhydrous silica previously sieved thought a sieve of 600 µm was added to the mixture and was blended for 3 minutes at 13 rpm. Finally, the magnesium stearate previously sieved thought a sieve of 600 µm was added to the mixture and blended for 3 minutes at 13 rpm. The lubricated mixture was compressed to provide tablets of 150 mg of free Ibandronic acid as monohydrate Ibandronate sodium salt.

Tablets were compressed on a Fette 102i rotary tablet press. The rotor speed was 50.000 tablets per hour, the fill-o-matik speed was 25 rpm, and the compression force was about 11.5 KN (11.5KN = 180.55 MPa). Obtained tablets had a median hardness of 141.4N and 0.03% of friability.

### Process for manufacturing film-coating tablets

The film-coating suspension was prepared by blending PEG 6000 in water until dissolution. Titanium dioxide was added to the obtained dissolution and blended until homogenization. Finally, hydroxypropylcellulose (HPC) was added to the mixture and blended also until homogenization.

The film-coating were performed using a Manesty XL Lab 01 coater, with a tablet charge of 900 g and a feed air temperature of 50 °C. The sprayed flow rate is 9.4 g/min. The final total weight of obtained tablets is 458.5 mg and the increase percentage of tablets weight is 2%.

### Comparative Example 1: Dissolution profile.

The target dissolution profile requires that more than 60% of free Ibandronic acid is dissolved up to 5 minutes and more than 85% up to 15 minutes.

| Time | A | B | C | D | E |
|---|---|---|---|---|---|
| 0 | 0 | 0 | 0 | 0 | 0,0 |
| 5 | 61,6 | 71,0 | 45,7 | 65,6 | 47,6 |
| 10 | 88,4 | 94,1 | 72,5 | 82,6 | 81,5 |
| 15 | 89,1 | 97,6 | 77,1 | 85,6 | 88,9 |
| 30 | 89,7 | 97,3 | 81,3 | 88,3 | 93,1 |
| 45 | 90,3 | 96,6 | 83,8 | 88,7 | 97,1 |

Pharmaceutical composition A comprises Avicel PH 102 and 15% croscarmellose.
Comparative pharmaceutical composition B is Bonviva® (Reference of dissolution profile required) 150mg Lot. B1064 in water.
Comparative pharmaceutical composition C comprises Avicel PH 102 and 5% croscarmellose.
Pharmaceutical composition D comprises Avicel PH 102 and 10% croscarmellose.
Comparative pharmaceutical composition E comprises comprecel 200 and 10% croscarmellose.

The dissolution profile results show that the pharmaceutical composition of the invention (A and D) have the required dissolution profile as Bonviva. Thus, more than 60% of free Ibandronic acid is dissolved up to 5 minutes and more than 85% up to 15 minutes.

Comparative pharmaceutical composition C (having 5% croscarmellose, outside the claimed range) gives rise to a different dissolution profile, which is considered not appropriate. Thus, at 5 minutes only a 45.7% of free Ibandronic acid is dissolved and at 15 minutes only a 77.1% of free Ibandronic acid is dissolved. Comparative pharmaceutical composition E (using microcrystalline cellulose having a median particle size outside the claimed range) gives rise also to a different dissolution profile. Thus, at 5 minutes only a 47.6% of free Ibandronic acid is dissolved.

### Example 3: Stability data test

Three-month data test stability of batch number DRODG0801 and DRODG0802 of tablets of monohydrate Ibandronate sodium salt packaged in an Aluminum/Triplex blister and in a Aluminium/Aluminum blisters. The results are shown in Table 1.

**Table 1:**

| **Conditions** | | **Assay (%)** | | | |
|---|---|---|---|---|---|
| Temperature (°C) | Relative Humidity (%) | DRODG0801 | | DRODG0802 | |
| | | TIME 0 | 3 MONTHS | TIME 0 | 3 MONTHS |
| 25^{a} | 60 | 98.0 | 99.4 | 97.0 | 98.5 |
| 30^{a} | 65 | 98.0 | 97.5 | 97.0 | 99.7 |
| 40^{a} | 75 | 98.0 | 98.9 | 97.0 | 98.8 |
| 30^{b} | 65 | 98.0 | 97.4 | 97.0 | 97.9 |
| 40^{b} | 75 | 98.0 | 98.3 | 97.0 | 98.9 |

| | | | | | |
|---|---|---|---|---|---|
| a: Aluminium/Triplex blisters b: Aluminium/Aluminium blisters | | | | | |

The analytical method used to determine the percentage of monohydrate ibandronate sodium salt (assay) in the tablets of the present invention was high performance liquid chromatography (HPLC) with an index refraction detector.

The chromatographic conditions of the analysis were as follows:

| | |
|---|---|
| Column | Sunfire C-18; 150 mm x 4.6 mm, 3.5µm |
| Volume of injection | 50 µl |
| Flux | 1,25 ml/min |
| Column temperature | 40°C |
| Injector temperature | 40°C |
| Detector temperature | 40°C |
| Polarity | Positive |
| Mobil phase | Acetonitrile : buffer 0.1% Trifluoroacetic acid |
| | (4: 96 v/v) |
| Time of analysis | 8 min |

The content (%) of purity at 3 months and time zero ([T3-T0] are shown in Table 2:

**Table 2:**

| **Conditions** | | **Assay (%)** | |
|---|---|---|---|
| Temperature (°C) | Relative Humidity (%) | DRODG0801 | DRODG0802 |
| | | \|T3-T0\| | \|T3-T0\| |
| 25^{a} | 60 | +1,4 | +1,5 |
| 30^{a} | 65 | +0,5 | +2,7 |
| 40^{a} | 75 | +0,9 | +1,8 |
| 30^{b} | 65 | -0,6 | +0,9 |
| 40^{b} | 75 | +0,3 | +1,9 |

The discrepancies of the content of monohydrate ibandronate sodium salt after the stability study at 3 months were lower than 3.0% and only one of the obtained values was lower than in time zero. The variability of the results was due to the inherent sensibility of the index refraction detector of the HPLC equipment.

Thus, the previous results show that the active ingredient is stable in the pharmaceutical compositions of the present invention, both when the pharmaceutical composition as a tablet is packaged in an Aluminium/Triplex blisters and when is packaged in an Aluminium/Aluminium blisters. The active ingredient in the pharmaceutical compositions of the present invention is also stable when a less protective packaging material is used, such as Triplex.

## Claims

1. A pharmaceutical composition which comprises from 0.5 to 40% by weight of Ibandronate sodium salt or a hydrate thereof, from 15 to 30% by weight of microcrystalline cellulose with a median particle size (D50) from 30 to 150 µm and from 10 to 15% by weight of croscarmellose sodium.

2. The pharmaceutical composition according to claim 1, which comprises 36% by weight of the Ibandronate sodium salt or a hydrate thereof.

3. The pharmaceutical composition according to claim 2, which is administered once per month.

4. The pharmaceutical composition according to any of the claims 1-3, wherein the median particle size (D50) of microcrystalline cellulose is 100 µm.

5. The pharmaceutical composition according to any of the claims 1-4, which comprises from 11-15% by weight of croscarmellose sodium.

6. The pharmaceutical composition according to claim 5, which comprises from 12-15% by weight of croscarmellose sodium.

7. The pharmaceutical composition according to any of the claims 1-6, further comprising a diluent selected from the group consisting of anhydrous lactose and hydrous fast flow lactose.

8. The pharmaceutical composition according to claim 7, further comprising a lubricant and anti-adherent agents.

9. The pharmaceutical composition according to any of the claims 1-8, which comprises:
36.7% by weight of the Ibandronate sodium salt or a hydrate thereof;
25.3% by weight of microcrystalline cellulose;
14.7% by weight of croscarmellose sodium;
19.3% by weight fast flow lactose;
1 % by weight of magnesium stearate; and
1 % by weight of colloidal anhydrous silica.

10. The pharmaceutical composition according to any of the claims 1 to 9, which is a tablet.

11. The pharmaceutical composition according to claim 10, wherein the tablet is a film-coating tablet.

12. The pharmaceutical composition according to claim 11, wherein the film-coating comprises hydroxypropylcellulose, titanium dioxide, and polyethylenglycol 6000.

13. A process for the preparation of a pharmaceutical composition as defined in any of the claims 1-12, which comprises:
(a) mixing Ibandronate sodium salt or a hydrate thereof with microcrystalline cellulose, a diluent, and croscarmellose sodium;
(b) incorporating a lubricant to the mixture of step (a); and
(c) incorporating an anti-adherent to the mixture of step (b).

14. The process according to claim 13, wherein the Ibandronate sodium salt or a hydrate thereof is previously sieved through a sieve of 813 µm, the lubricant and the anti-adherent are sieved through a sieve of 600 µm.

15. The process according to any of the claims 13-14, further comprising a direct compression of the mixture of step (c) to obtain a tablet.
